# EUROPEAN PATENT APPLICATION

(11) **EP 4 261 273 A1**
(43) Date of publication of application: **18.10.2023**
(21) Application number: 22736921.2
(22) Date of filing: 11.01.2022
(51) Int. Cl.: C12N 5/00, C12N 5/071, C12M 1/12, C12N 5/077

(54) **SCAFFOLD DERIVED FROM DECELLULARIZED ADIPOSE TISSUE FOR CULTURING ORGANOID, AND METHOD FOR PRODUCING SAME**

(30) Priority: 11.01.2021 KR 20210003144
(71) Applicant: Cellartgen Inc., Seoul 03722 (KR)
(72) Inventor: CHO, Seung Woo, Seoul 03722 (KR); YUN, In Sik, Seoul 06273 (KR); CHOI, Yi Sun, Seoul 03722 (KR); KIM, Su Ran, Seoul 03722 (KR); MIN, Sung Jin, Seoul 03722 (KR); KIM, Su Kyeom, Seoul 03722 (KR); KIM, Yu Heun, Seoul 03722 (KR); PARK, Se Won, Seoul 03722 (KR)
(74) Representative: Manitz Finsterwald Patent- und Rechtsanwaltspartnerschaft mbB
(86) International application number: PCT/KR2022/000429
(87) International publication number: WO 2022/149944

(57) **Abstract**

The present disclosure relates to a decellularized adipose tissue-derived scaffold and a method of preparing the same. The scaffold of the present disclosure can be applied to culture of various types of organoids and thus can be used in various fields due to its high versatility. Also, the scaffold of the present disclosure can be prepared from a human adipose tissue to be discarded and thus can create enormous added value. Further, the scaffold of the present disclosure can be widely used in the medical industry, such as new drug development, drug toxicity and efficacy evaluation, and patient-specific drug selection, in substitution for conventional Matrigel.

## Description

### TECHNICAL FIELD

The present disclosure relates to a decellularized adipose tissue-derived scaffold for organoid culture and a method of preparing the same.

### BACKGROUND

A conventional two-dimensional (2D) cell culture method is limited in implementing an in vivo microenvironment and thus has a low culture efficiency. Therefore, a 2D cell line is a limited in vitro model. A three-dimensional organoid culture technology has recently attracted a lot of interest as a new alternative to overcome these limitations. The organoid is a technology that has been rapidly growing worldwide and can be applied as a tissue analogue to various application fields, such as drug screening, drug toxicity evaluation, disease modeling and cell therapeutic agent. The organoid is a three-dimensional structure composed of various cells of a specific human organ and tissue, and can implement complicated interactions among them. Therefore, it can be applied as a more accurate in vitro model platform than conventionally used drug evaluation models such as 2D cell line models or animal models.

Platforms for organoids derived from various organs have been established worldwide and relevant research is still being actively conducted. Currently, Matrigel is used as a culture scaffold to culture various types of organoids.

However, since Matrigel is an extracellular matrix component extracted from a mouse sarcoma tissue, it is difficult to maintain the quality of products uniformly and it is expensive and has a safety problem such as transmission of animal pathogens and viruses. Therefore, Matrigel as an organoid culture system has a lot of problems to be solved. In particular, as a material derived from a cancer tissue, it cannot provide an optimal tissue-specific microenvironment necessary for culturing a specific tissue organoid.

There have been some studies on the development of polymer-based hydrogels to replace Matrigel, but no material has been reported that can replace Matrigel.

Meanwhile, hundreds of tons of human adipose tissues are discarded every year. Thus, it is expected that it can be utilized as a matrix material for culturing organoids with high economic feasibility if used well. Also, it is expected that a versatile scaffold capable of culturing various organoids regardless of the types of organs can create huge economic profits.

In the present disclosure, a hydrogel matrix composed of adipose tissue-specific extracellular matrix components was prepared through a decellularization process of adipose tissue and applied to organoid culture. In the present disclosure compared with the conventional methods for organ decellularization, a decellularization process includes a process of cutting the raw tissue into small chunks. Thus, the cells can be more effectively removed and the extracellular matrix components and growth factors abundantly present in the human adipose tissue can be well preserved. Therefore, it is confirmed that the hydrogel matrix can induce efficient growth and differentiation of various types of organoids and thus can replace the conventional Matrigel as a culture matrix with high versatility.

### DISCLOSURE OF THE INVENTION

### PROBLEMS TO BE SOLVED BY THE INVENTION

The present disclosure is conceived to provide a scaffold for the culture and transplantation of an organoid including an adipose extracellular matrix (AEM).

The present disclosure is also conceived to provide a method of preparing a scaffold for the culture and transplantation of an organoid, including: a process (1) of chopping an isolated adipose tissue; and a process (2) of treating the chopped adipose tissue with Triton X-100 and ammonium hydroxide for decellularization to prepare a decellularized AEM.

The present disclosure is further conceived to provide a method of culturing an organoid in the above-described scaffold or in a scaffold prepared by the above-described preparation method.

### Means for solving the problems

An aspect of the present disclosure provides a scaffold for culture and transplantation of an organoid including an adipose extracellular matrix (AEM).

In an embodiment of the present disclosure, the AEM may be prepared by using a mixed solution of Triton X-100 and ammonium hydroxide.

In an embodiment of the present disclosure, a concentration of the AEM in the scaffold may be from 1 mg/ml to 10 mg/ml.

Another aspect of the present disclosure provides a method of preparing a scaffold for culture and transplantation of an organoid, including a process (1) of crushing an isolated adipose tissue; and a process (2) of treating the crushed adipose tissue with Triton X-100 and ammonium hydroxide for decellularization to prepare a decellularized AEM.

In an embodiment of the present disclosure, the method may further include, after the process (2), a process (3) of lyophilizing the decellularized AEM to prepare a lyophilized AEM.

In an embodiment of the present disclosure, the method may further include, after the process (3), a process (4) of forming a scaffold for the culture and transplantation of an organoid in the form of a hydrogel with the lyophilized AEM.

In an embodiment of the present disclosure, in the process (4), the lyophilized AEM may be dissolved in a pepsin solution and the pH of the solution may be adjusted to form the hydrogel.

Yet another aspect of the present disclosure provides a method of culturing an organoid in the above-described scaffold or a scaffold prepared by the above-described preparation method.

### Effects of the invention

Since the scaffold according to the present disclosure enables efficient culture of organoids, it is expected to be widely used in the medical industry, such as new drug development, drug toxicity and efficacy evaluation, and patient-specific drug selection, in substitution for the conventional Matrigel, which has various problems. Accordingly, it is expected to improve the quality of life of people in terms of health care and create high added value in industrial and economic terms.

The scaffold developed in the present disclosure can be applied to culture of various types of organoids and thus can be used in various fields due to its high versatility. Also, the scaffold developed in the present disclosure is prepared from a human adipose tissue to be discarded and thus is expected to create enormous added value.

### BRIEF DESCRIPTION OF THE DRAWINGS

**FIG. 1A** is a schematic diagram showing a process of preparing a decellularized adipose extracellular matrix (AEM).
**FIG.1B** is a schematic diagram showing a process of preparing a decellularized adipose extracellular matrix (AEM).
**FIG. 1C** is a schematic diagram showing a process of preparing a decellularized adipose extracellular matrix (AEM).
**FIG. 2A** shows the results of analyzing the decellularized AEM for organoid culture.
**FIG. 2B** shows the results of analyzing the decellularized AEM for organoid culture.
**FIG. 2C** shows the results of analyzing the decellularized AEM for organoid culture.
**FIG. 3** shows the results of evaluating biocompatibility of the decellularized AEM.
**FIG. 4** shows the results of evaluating biocompatibility of the decellularized AEM.
**FIG. 5A** shows the results of proteomics of the decellularized AEM.
**FIG. 5B** shows the results of proteomics of the decellularized AEM.
**FIG. 5C** shows the results of proteomics of the decellularized AEM.
**FIG. 5D** shows the results of proteomics of the decellularized AEM.
**FIG. 6A** shows the results of proteomics of the decellularized AEM.
**FIG. 6B** shows the results of proteomics of the decellularized AEM.
**FIG. 6C** shows the results of proteomics of the decellularized AEM.
**FIG. 7A** shows the results of proteomics of the decellularized AEM.
**FIG. 7B** shows the results of proteomics of the decellularized AEM.
**FIG. 7C** shows the results of proteomics of the decellularized AEM.
**FIG. 8A** shows the results of analyzing physical properties depending on the concentration of the decellularized AEM.
**FIG. 8B** shows the results of analyzing physical properties depending on the concentration of the decellularized AEM.
**FIG. 9A** shows the results of selecting an optimal concentration of a decellularized AEM hydrogel for intestinal (small intestinal) organoid culture.
**FIG. 9B** shows the results of selecting an optimal concentration of a decellularized AEM hydrogel for intestinal (small intestinal) organoid culture.
**FIG. 10A** shows the results of comparing intestinal (small intestinal) organoid culture patterns depending on the concentration of the decellularized AEM hydrogel.
**FIG. 10B** shows the results of comparing intestinal (small intestinal) organoid culture patterns depending on the concentration of the decellularized AEM hydrogel.
**FIG. 11A** shows the results of selecting an optimal concentration of the decellularized AEM hydrogel for lung organoid culture.
**FIG. 11B** shows the results of selecting an optimal concentration of the decellularized AEM hydrogel for lung organoid culture.
**FIG. 12A** shows the results of comparing lung organoid culture patterns depending on the concentration of the decellularized AEM hydrogel.
**FIG. 12B** shows the results of comparing lung organoid culture patterns depending on the concentration of the decellularized AEM hydrogel.
**FIG. 13A** shows the results of selecting an optimal concentration of the decellularized AEM hydrogel for pancreatic organoid culture.
**FIG. 13B** shows the results of selecting an optimal concentration of the decellularized AEM hydrogel for pancreatic organoid culture.
**FIG. 14A** shows the results of comparing pancreatic organoid culture patterns depending on the concentration of the decellularized AEM hydrogel.
**FIG. 14B** shows the results of comparing pancreatic organoid culture patterns depending on the concentration of the decellularized AEM hydrogel.
**FIG. 15A** shows the results of selecting an optimal concentration of the decellularized AEM hydrogel for gastric organoid culture.
**FIG. 15B** shows the results of selecting an optimal concentration of the decellularized AEM hydrogel for gastric organoid culture.
**FIG. 16A** shows the results of comparing gastric organoid culture patterns depending on the concentration of the decellularized AEM hydrogel.
**FIG. 16B** shows the results of comparing gastric organoid culture patterns depending on the concentration of the decellularized AEM hydrogel.
**FIG. 17A** shows the results of selecting an optimal concentration of the decellularized AEM hydrogel for kidney organoid culture.
**FIG. 17B** shows the results of selecting an optimal concentration of the decellularized AEM hydrogel for kidney organoid culture.
**FIG. 18A** shows the results of comparing kidney organoid culture patterns depending on the concentration of the decellularized AEM hydrogel.
**FIG. 18B** shows the results of comparing kidney organoid culture patterns depending on the concentration of the decellularized AEM hydrogel.
**FIG. 19A** shows the results of selecting an optimal concentration of the decellularized AEM hydrogel for hepatic (biliary) organoid culture.
**FIG. 19B** shows the results of selecting an optimal concentration of the decellularized AEM hydrogel for hepatic (biliary) organoid culture.
**FIG. 20** shows the results of comparing hepatic (biliary) organoid culture patterns depending on the concentration of the decellularized AEM hydrogel.
**FIG. 21** shows the results of selecting an optimal concentration of the decellularized AEM hydrogel for esophageal organoid culture.
**FIG. 22A** shows the results of comparing esophageal organoid culture patterns depending on the concentration of the decellularized AEM hydrogel.
**FIG. 22B** shows the results of comparing esophageal organoid culture patterns depending on the concentration of the decellularized AEM hydrogel.
**FIG. 23** shows the results of intestinal (colonic) organoid culture using the decellularized AEM hydrogel.
**FIG. 24** shows the results of selecting an optimal concentration of the decellularized AEM hydrogel for human-induced pluripotent stem cell (hiPSC)-derived hepatic organoid culture.
**FIG. 25** shows the results of comparing hiPSC-derived hepatic organoid patterns depending on the concentration of the decellularized AEM hydrogel.
**FIG. 26A** shows the results of cardiac organoid culture using the decellularized AEM hydrogel.
**FIG. 26B** shows the results of cardiac organoid culture using the decellularized AEM hydrogel.
**FIG. 27A** shows the results of analyzing the functionality of the cardiac organoid prepared in the decellularized AEM hydrogel.
**FIG. 27B** shows the results of analyzing the functionality of the cardiac organoid prepared in the decellularized AEM hydrogel.
**FIG. 27C** shows the results of analyzing the functionality of the cardiac organoid prepared in the decellularized AEM hydrogel.

### BEST MODE FOR CARRYING OUT THE INVENTION

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operations and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless otherwise defined herein, all technical and scientific terms used herein have the same meaning as commonly understood by a person with ordinary skill in the art to which this disclosure belongs.

Various scientific dictionaries that include the terms included herein are well-known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

In the present disclosure, a process of preparing a decellularized scaffold from a large amount of human adipose tissue through a series of chemical and physical treatments was developed, and this process was successfully used to culture various types of organoids.

A decellularized adipose tissue-derived scaffold developed in the present disclosure is composed of major extracellular matrix components with cells removed. Thus, it was expected to have excellent biocompatibility without causing an immune response when transplanted into the body. It was actually confirmed from proteomics that the decellularized adipose tissue-derived scaffold abundantly contains various extracellular matrix components and related proteins. Therefore, it is possible to form, develop and maintain various organoids in the developed decellularized adipose tissue-derived scaffold.

According to the present disclosure, it was actually confirmed that various types of organoids were formed and grown using a decellularized adipose tissue-derived hydrogel scaffold prepared in the present disclosure. Also, an optimal concentration for each organoid culture was selected by testing various extracellular matrix scaffold concentrations.

As a result, it was confirmed that various types of organoids can be cultured in similar patterns in the decellularized adipose tissue-derived scaffold and can also be differentiated into tissue cells constituting each organ, which verifies that the decellularized adipose tissue-derived scaffold can replace the conventional Matrigel as a culture matrix. Also, it was confirmed that the scaffold of the present disclosure can be applied as a versatile matrix for organoid culture regardless of the types of organs.

An aspect of the present disclosure provides a scaffold for culture and transplantation of an organoid including an adipose extracellular matrix (AEM).

The term "extracellular matrix" refers to a natural scaffold for cell growth that is prepared by decellularization of tissue found in mammals and multicellular organisms. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various tissues may differ in their overall structure and composition due to the unique role needed for each tissue.

The term "derive" or "derived" refers to a component obtained from any stated source by any useful method.

In an embodiment of the present disclosure, the AEM may be prepared by using a mixed solution of Triton X-100 and ammonium hydroxide.

In an embodiment of the present disclosure, a concentration of the AEM in the scaffold may be from 1 mg/ml to 10 mg/ml, specifically from 2 mg/ml to 7 mg/ml. The concentration of the AEM may be, for example, from 2 mg/ml to 7 mg/ml, from 2 mg/ml to 6 mg/ml, from 2 mg/ml to 5 mg/ml, from 2 mg/ml to 4 mg/ml, from 2 mg/ml to 3 mg/ml, from 3 mg/ml to 7 mg/ml, from 3 mg/ml to 6 mg/ml, from 3 mg/ml to 5 mg/ml, from 3 mg/ml to 4 mg/ml, from 4 mg/ml to 7 mg/ml, from 4 mg/ml to 6 mg/ml, from 4 mg/ml to 5 mg/ml, from 5 mg/ml to 7 mg/m, from 5 mg/ml to 6 mg/ml, or from 6 mg/ml to 7 mg/ml. In an example, the concentration of the AEM may be 1 mg/ml, 2 mg/ml, 3 mg/ml, 4 mg/ml, 5 mg/ml, 6 mg/ml, or 7 mg/ml. The AEM contained with a concentration out of the above-described range may make it impossible to achieve the intended effect of the present disclosure.

More specifically, the concentration of the AEM may be determined depending on the type of an organoid to be cultured, and may be, for example, 4 mg/ml for a small intestinal organoid, 7 mg/ml for a lung organoid, 5 mg/ml for a pancreatic organoid, 7 mg/ml for a gastric organoid, 5 mg/ml for a kidney organoid, 3 mg/ml for a tissue-derived hepatic (biliary) organoid, 5 mg/ml for a colonic organoid, 7 mg/ml for a cardiac organoid, and 7 mg/ml for a human induced pluripotent stem cell (hiPSC)-derived hepatic organoid.

The scaffold includes a three-dimensional hydrogel prepared based on the AEM obtained by decellularization, and can be effectively used for organoid culture.

The decellularized adipose tissue contains extracellular matrix components capable of enhancing proliferation, differentiation and functionality of various cells and thus is highly efficient in enhancing growth, development and functionality of an organoid.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the intrinsic physiological properties of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions and to have an organ-like form with functionality and a tissue-specific function.

The scaffold of the present disclosure contains the AEM, which has high versatility compared with other tissue-derived extracellular matrices, and thus can be used to culture various organoids. The organoid to be cultured may be any one of a small intestinal organoid, a lung organoid, a pancreatic organoid, a gastric organoid, a kidney organoid, a hepatic organoid, an esophageal organoid, a biliary organoid, a colonic organoid and a cardiac organoid.

Another aspect of the present disclosure provides a method of preparing a scaffold for culture and transplantation of an organoid, including a process (1) of chopping an isolated adipose tissue; and a process (2) of treating the crushed adipose tissue with Triton X-100 and ammonium hydroxide for decellularization to prepare a decellularized AEM.

The process (1) is a process of crushing an isolated adipose tissue, and the adipose tissue may be isolated from a known animal. Examples of the animal may include cattle, pigs, monkeys, humans, etc. Also, in the present disclosure, the isolated adipose tissue is crushed and then decellularized, which results in high efficiency in decellularization. The isolated adipose tissue may be crushed by a known method. According to the present disclosure, the adipose tissue was crushed and decellularized, and, thus, the cells can be removed more efficiently at a higher level.

The process (2) is a process of treating the crushed adipose tissue with Triton X-100 and ammonium hydroxide for decellularization to prepare a decellularized AEM. According to the present disclosure, the tissue is treated with Triton X-100 and ammonium hydroxide to minimize damage to the tissue, and, thus, it is possible to preserve various proteins more in the adipose tissue. For example, the decellularization may be performed by stirring the crushed adipose tissue with Triton X-100 and ammonium hydroxide, followed by stirring with Dnase I (2000 KU) for 3 hours and isopropanol for 36 hours.

In an embodiment of the present disclosure, the method may further include, after the process (2), a process (3) of lyophilizing the decellularized AEM to prepare a lyophilized AEM.

The process (3) is a process of lyophilizing the decellularized AEM to prepare a lyophilized AEM. After drying, the lyophilized AEM may be exposed to electron beam, gamma radiation, ethylene oxide gas, or supercritical carbon dioxide for sterilization.

In an embodiment of the present disclosure, the method may further include, after the process (3), a process (4) of forming a scaffold for culture and transplantation of an organoid in the form of a hydrogel with the lyophilized AEM.

The process (4) is a process of forming a scaffold for culture and transplantation of an organoid in the form of a hydrogel with the lyophilized AEM. The process (4) may be performed through gelation. Specifically, the lyophilized AEM may be dissolved in a pepsin solution and a pH of the solution may be adjusted to form a hydrogel. The decellularized AEM may be crosslinked to prepare a three-dimensional hydrogel-type scaffold, and the gelated scaffold can be used in various ways in the fields related to tests, screening and organoid culture.

The term "hydrogel" is a material in which a liquid that contains water as a dispersion medium is hardened through a sol-gel phase transition to lose fluidity and form a porous structure. The hydrogel can be formed by causing a hydrophilic polymer that has a three-dimensional network structure and a microcrystalline structure to contain water and be expanded.

The gelation may be performed at a temperature of 37°C for 30 minutes after dissolving the lyophilized AEM in an acidic solution with a protease such as pepsin or trypsin and adjusting a pH, specifically setting a neutral pH in an electrolyte state of 1X PBS buffer by using 10X PBS and 1 M NaOH.

Yet another aspect of the present disclosure provides a method of culturing an organoid in the above-described scaffold or a scaffold prepared by the above-described preparation method.

The conventional Matrigel-based culture system is an extract derived from an animal cancer tissue, has a large difference between the batches, cannot mimic a microenvironment of an actual tissue, and exhibits insufficient efficiency in differentiation or development into an organoid. However, the scaffold can create a tissue-like environment and thus is suitable for culturing various organoids.

The culture refers to a process of maintaining and growing cells under suitable conditions, and the suitable conditions may refer to, for example, the temperature, nutrient availability, atmospheric CO₂ level and cell density at which the cells are maintained.

Appropriate culture conditions for maintaining, proliferating, expanding and differentiating different types of cells are known in the art and are documented. Suitable conditions for formation of the organoid may facilitate or allow cell differentiation and formation of a multicellular structure.

### Mode for Carrying out the Invention

Hereafter, one or more embodiments will be described in more detail with reference to one or more examples. However, these examples serve to explain one or more embodiments by way of example, and the scope of the present disclosure is not limited to these examples.

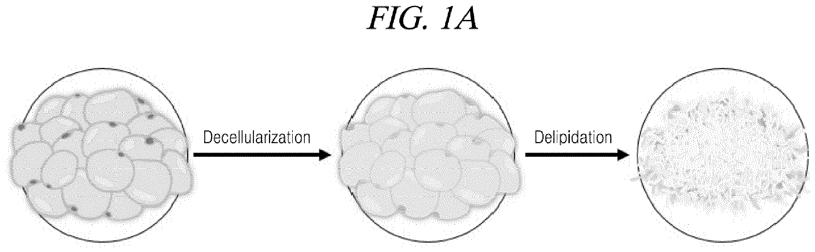

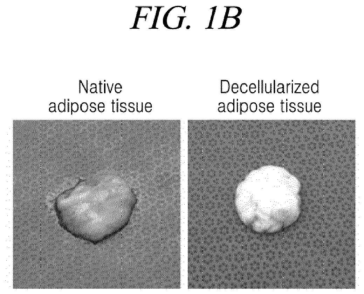

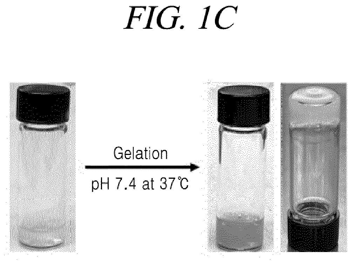

### Preparation of decellularized adipose extracellular matrix (AEM) (FIG. 1A, FIG. 1B, FIG. 1C

Cells were removed from a human adipose tissue through decellularization to prepare an AEM scaffold composed of an extracellular matrix. In the decellularization process used in the present disclosure, the human adipose tissue was cut into small chunks or fat sucked by means of a negative pressure was collected, followed by decellularization. Thus, it is possible to more effectively remove the cells.
(A) A schematic diagram showing a process of preparing a decellularized AEM scaffold for organoid culture
(B) A decellularized human adipose tissue was prepared by applying physical stimuli through a series of chemical treatments (continuous stirring with 1 M sodium chloride (NaCl) for 2 hours at 37°C, 1% Triton X-100 and 0.1% ammonium hydroxide (NH₄OH) for 18 hours at room temperature, Dnase I (2000 KU) for 3 hours and isopropanol for 36 hours) to a native human adipose tissue and effectively removing cells, followed by lyophilization to obtain an AEM.
(C) 10 mg of the lyophilized AEM was treated with 1 ml of a 6 mg/ml pepsin solution (a solution of 6 mg of pepsin powder derived from porcine gastric mucosa in 1 ml of 0.02 M HCl) and stirred at room temperature for 48 hours to carry out a solution process. After 10X PBS and NaOH were added into the prepared AEM solution to adjust the pH and electrolyte concentration suitable for cell culture, the formation of a hydrogel was induced at 37°C for 30 minutes.

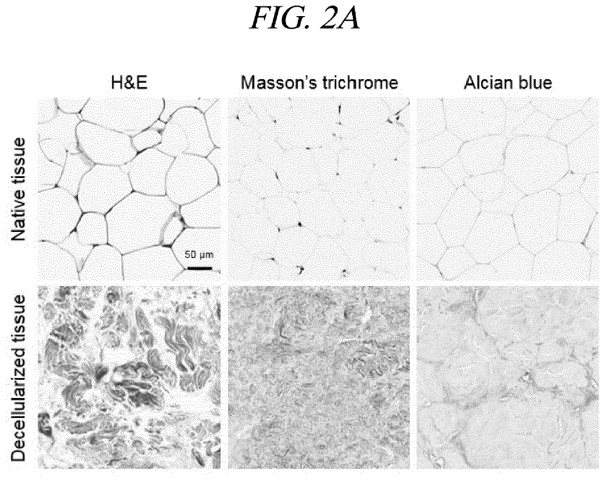

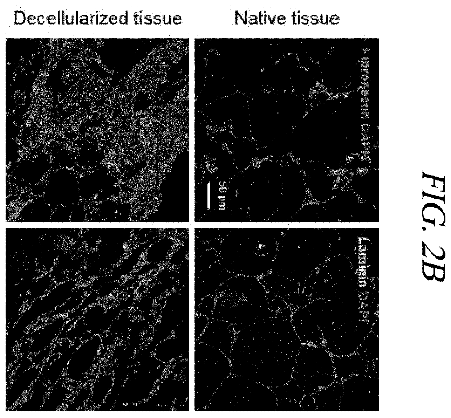

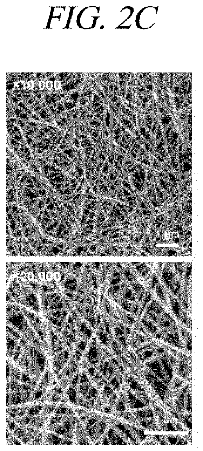

### Analysis of decellularized AEM for organoid culture (FIG. 2A, FIG. 2B, FIG. 2C)

(A) Hematoxylin & eosin staining was performed on a human adipose tissue before (Native tissue) and after (Decellularized tissue) decellularization. As a result, all of the cells were removed and only the extracellular matrix components remained after the decellularization process. Also, Masson's trichrome staining was performed to confirm that the collagen components were well preserved in the decellularized AEM. Further, Alcian blue staining was performed to confirm that the glycosaminoglycan components were well preserved and maintained.
(B) Also, immunostaining was performed to check whether major proteins that play an important role in the extracellular matrix components, such as fibronectin and laminin, are well preserved in the adipose tissue after the decellularization process. It was confirmed that fibronectin and laminin were well maintained in the AEM. Further, the nuclei stained with DAPI were observed to disappear after the decellularization process, which confirmed that the cell components were removed.
(C) A microstructure inside the three-dimensional AEM hydrogel was analyzed using a scanning electron microscope (SEM). As a result, the AEM hydrogel was observed to have a nanofiber-based microporous internal structure. Therefore, the AEM hydrogel was expected to provide a three-dimensional microenvironment suitable for culturing various organoids.

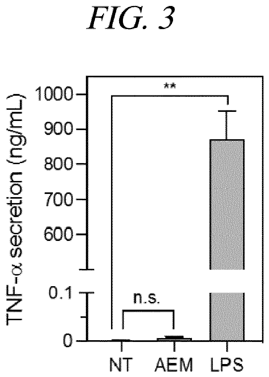

### Evaluation of biocompatibility of decellularized AEM-1 (in vitro) (FIG. 3)

To evaluate the effect of the decellularized AEM on immune cells, an AEM hydrogel was cultured with macrophages (Raw 264.7) and the amount of inflammatory cytokine TNF-α (tumor necrosis factor) secreted by Raw 264.7 when an immune response was induced was measured by ELISA.

It was confirmed that the AEM hydrogel induced the secretion of TNF-α at an insignificant level similar to the case without any treatment. Thus, it was predicted that the AEM hydrogel would not induce an inflammatory response when applied to the body.

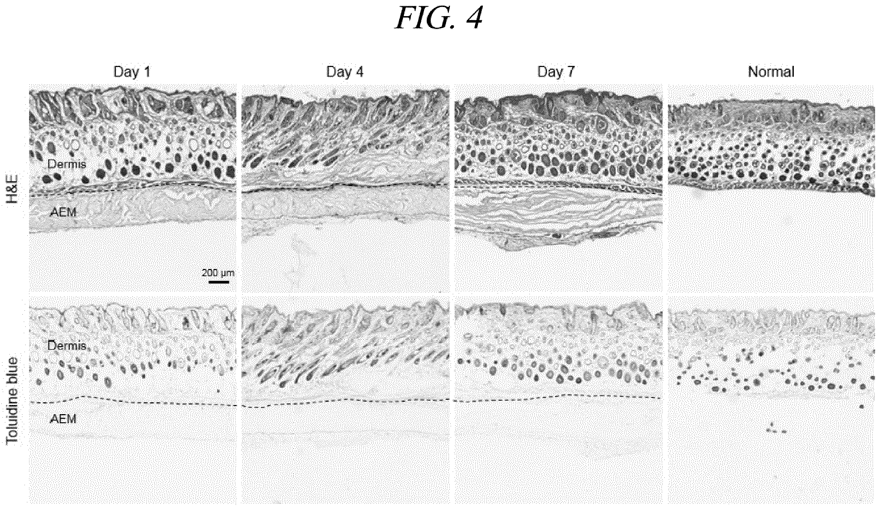

### Evaluation of biocompatibility of decellularized AEM-2 (in vivo) (FIG. 4)

To check the applicability of the decellularized AEM as a material for transplantation, an AEM hydrogel was transplanted subcutaneously into a mouse and then, the presence or absence of immune response and inflammatory response was checked for one week.

It was confirmed from H&E histological staining that an abnormal inflammatory response, such as tissue necrosis and infiltration of immune cells, did not occur at the site where the AEM hydrogel was transplanted. Also, toluidine blue staining, which stains the cytoplasm of mast cells reddish purple among immune cells, was performed to confirm that the mast cells were not found at the tissue site where the AEM hydrogel was transplanted.

That is, tissue damage or an immune response did not occur during AEM hydrogel transplantation, which confirmed that the AEM can be applied not only as a material for culture but also as a material for organoid transplantation.

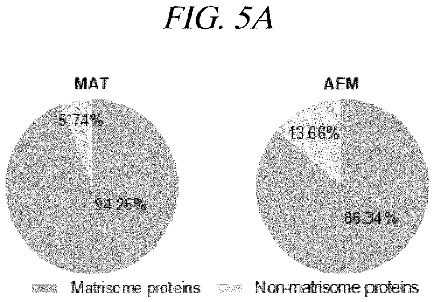

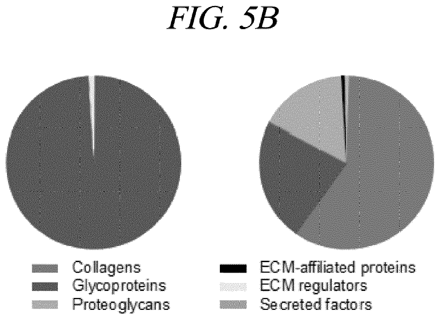

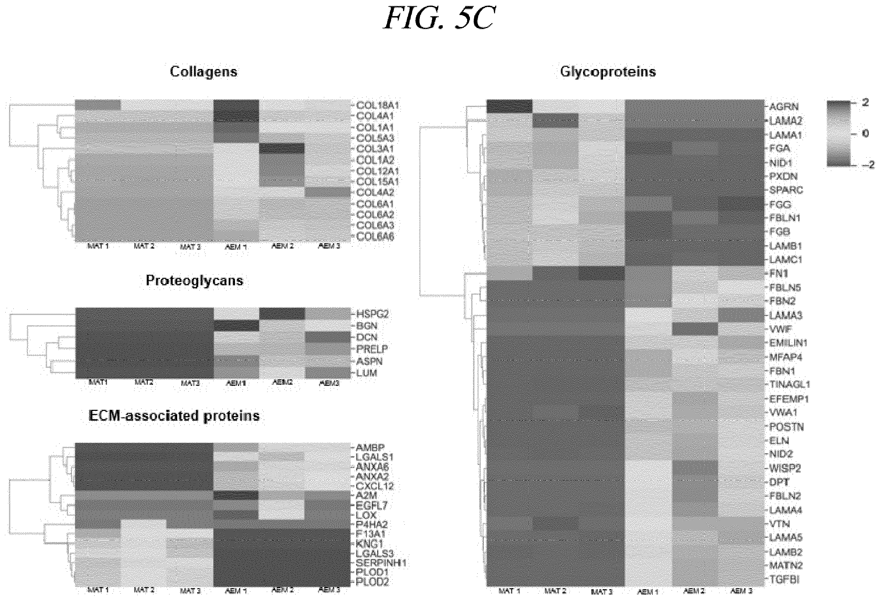

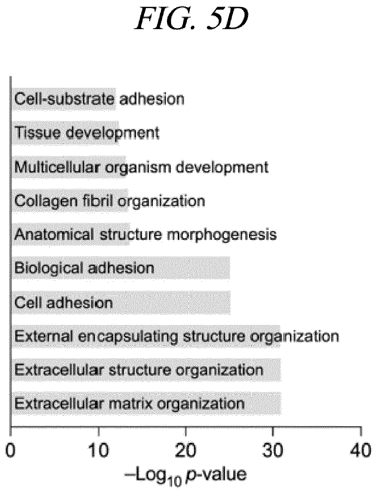

### Proteomics of decellularized AEM-1 (FIG. 5A, FIG. 5B, FIG. 5C, FIG. 5D)

To identify protein components contained in the decellularized human AEM, proteomics analysis was performed using a mass spectrometer.
(A) It was confirmed that the AEM is mostly composed of matrisome proteins and Matrigel (MAT), a control group, is also mostly composed of matrisome proteins.
(B) It was confirmed that the AEM contains various types of extracellular matrix components, such as collagen, glycoproteins and proteoglycans, and also contains various related proteins that regulate the extracellular matrix, whereas the MAT is mostly composed of glycoproteins.
(C) The difference in extracellular matrix proteins between the AEM and the MAT was analyzed through a heatmap. As a result, it was confirmed that the two scaffolds are greatly different in expression level distribution. In particular, various proteins in all of extracellular matrix categories were detected at high levels in the AEM, whereas the expression of most proteins except for glycoproteins and some extracellular matrix-related proteins was observed at low levels in the MAT.
(D) The proteins detected in a greater amount from the AEM than from the MAT were analyzed by gene ontology (GO). As a result, biological processes that play a major role in organoid formation, such as extracellular structure organization, extracellular matrix organization, collagen fibril organization and tissue development, were identified.

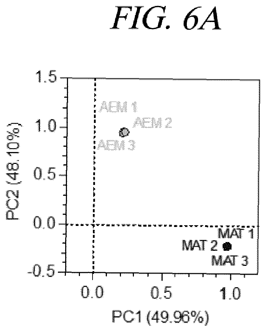

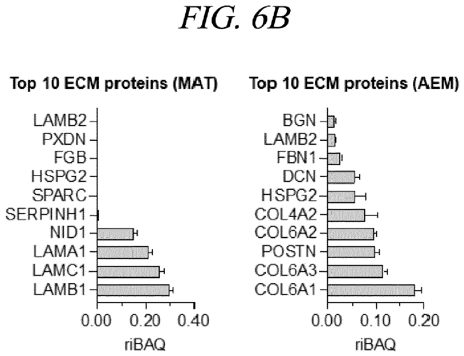

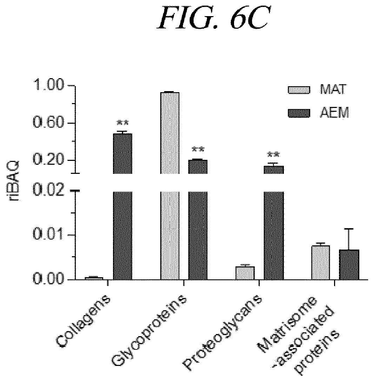

### Proteomics of decellularized AEM-2 (FIG. 6A, FIG. 6B, FIG. 6C)

(A) Principal component analysis (PCA) was performed to compare the degree of similarity between the AEM and the MAT. It was observed that AEM samples and MAT samples were far apart from each other in the analysis graph due to their low degree of similarity, which confirms that the AEM and the MAT are composed of significantly different components.
(B) Among ECM proteins detected in the decellularized AEM and the Matrigel (MAT), 10 proteins with the highest expression levels were selected, followed by quantitative analysis. It was confirmed that the Top 10 ECM proteins of the MAT are 8 glycoprotein proteins and 4 of them accounted for more than 90% of the MAT. However, it was confirmed that the Top 10 ECM proteins of the AEM consist of 4 collagen proteins, 3 glycoprotein proteins, and 3 proteoglycan proteins and are evenly distributed in the AEM.
(C) As a result of comparing the components of the AEM and the MAT, it was confirmed that the MAT is mostly composed of glycoproteins, whereas the AEM is evenly composed of collagens, glycoproteins, proteoglycans and other extracellular matrix-related proteins. This shows that the AEM can provide more diverse extracellular matrix microenvironments for organoid culture than the MAT.

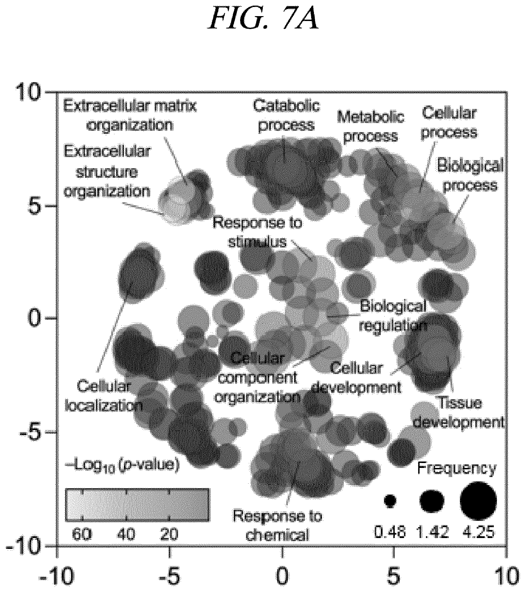

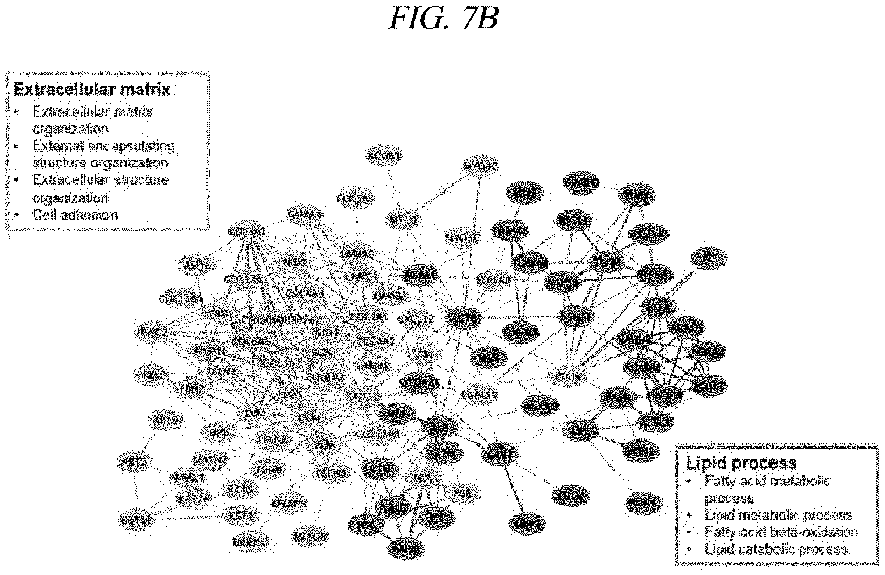

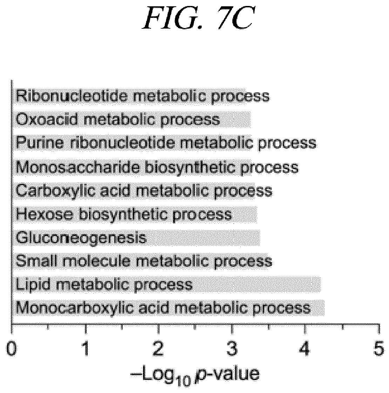

### Proteomics of decellularized AEM-3 (FIG. 7A, FIG. 7B, FIG. 7C)

(A) All the proteins extracted from the decellularized AEM were analyzed by gene ontology (GO). Then, the proteins with similar functions were clustered through a Multidimensional Scaling (MDS) algorithm. According to the gene ontology of the proteins constituting the AEM, it was predicted that biological processes related to these proteins would play an important role in the basic functions of cells and in particular, biological processes, such as extracellular matrix organization and metabolism, and cell and tissue development, would greatly contribute to efficient differentiation development of various organoids.
(B) All the proteins in the AEM were analyzed in terms of interactions among them and then clustered. Most of the proteins formed a network responsible for a biological process involved in the structure and composition of the extracellular matrix providing structural support and adhesion of cells, and the rest of the proteins formed a network related to lipid and energy metabolism. This is because the AEM is derived from human fat, and it is expected to be used for not only structural development, but also energy metabolism of various cells in organoid culture in the future.
(C) As a result of gene ontology of proteins that are expressed at least 4 times more in adipose tissue than in other tissues among the proteins detected from the AEM, it was confirmed that lipid metabolism- and adipocyte-related biological processes were identified.

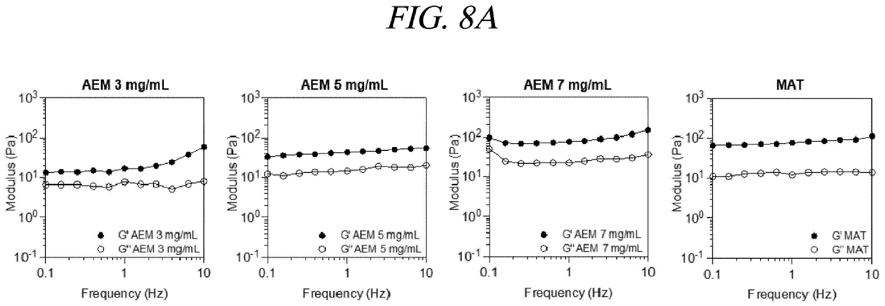

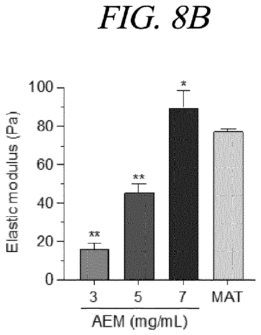

### Analysis of physical properties depending on concentration of decellularized AEM (FIG. 8A, FIG. 8B)

(A) To examine the differences in physical properties depending on the concentration of the decellularized AEM, hydrogels were formed at various concentrations (3 mg/ml, 5 mg/ml and 7 mg/ml) and then, an elastic modulus G' and a viscous modulus G" at a frequency in the range of 0.1 Hz to 10 Hz were measured with a rotary rheometer. It was confirmed that the elastic modulus representing the solid phase was observed to be higher than the viscous modulus representing the liquid phase in the entire frequency range, which shows that the internal structure of the AEM hydrogel is composed of a stable polymer network.
(B) The average elastic modulus at each concentration of the AEM was compared with that of the control group, Matrigel (MAT). It was confirmed that the mechanical properties improved as the AEM concentration increased. Also, it was confirmed that the AEM hydrogel at a concentration of 7 mg/ml had higher mechanical properties than the MAT.

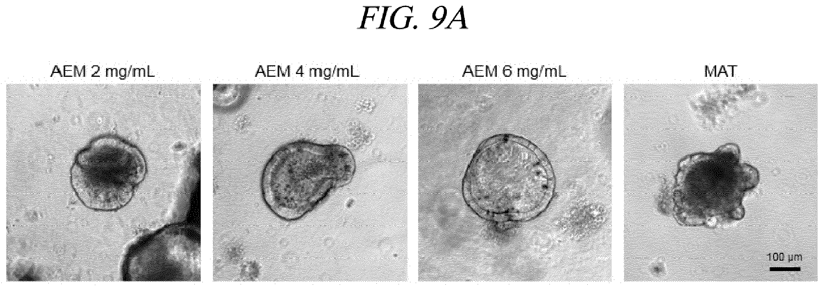

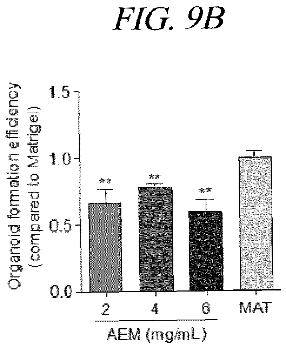

### Selection of optimal concentration of decellularized AEM hydrogel for intestinal (small intestinal) organoid culture (FIG. 9A, FIG. 9B)

To select an optimal hydrogel concentration when applying the decellularized AEM hydrogel to small intestinal organoid culture, a hydrogel was prepared at each AEM concentration, followed by small intestinal organoid culture. The Matrigel (MAT) was used as a control group. An upper part of the small intestine of a mouse was collected, and an intestinal crypt containing intestinal stem cells was isolated. Then, a crypt tissue was three-dimensionally cultured in the respective hydrogels to induce the formation of small intestinal organoids. On day 6 of culture, the small intestinal organoids formed at respective AEM concentrations were compared with the small intestinal organoid formed in the Matrigel in terms of morphology and formation efficiency.
(A) It was confirmed that the small intestinal organoids cultured in the AEM hydrogels at concentrations of 2 mg/ml, 4 mg/ml and 6 mg/ml, respectively, were formed with similar morphologies to that cultured in the Matrigel used as a control group.
(B) The small intestinal organoids cultured in the AEM hydrogels at different concentrations (2 mg/ml, 4 mg/ml and 6 mg/ml) and the Matrigel were compared in terms of formation efficiency. As a result, it was confirmed that the organoid formation efficiency was the highest at a concentration of 4 mg/ml.

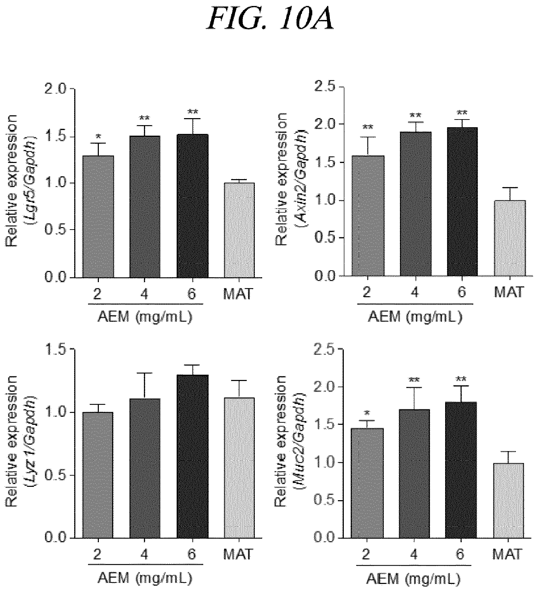

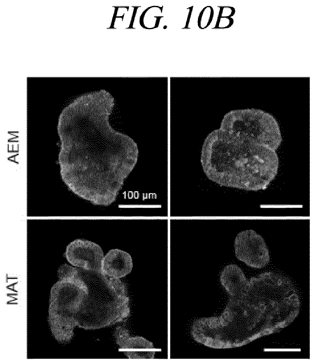

### Comparison of intestinal (small intestinal) organoid culture patterns depending on concentration of decellularized AEM hydrogel (FIG. 10A, FIG. 10B)

(A) As a result of comparing the gene expression levels of small intestinal organoids cultured for 6 days in the AEM hydrogels prepared at respective concentrations by quantitative PCR (qPCR) analysis, it was confirmed that Lgr5 and Axin2, stem ness-related genes, significantly increased in the AEM hydrogel group compared with the Matrigel group.

Also, Lyz1, a differentiation-related marker, showed no statistically significant difference between the AEM hydrogel group and the Matrigel group, and Muc2 showed a higher expression level in the small intestinal organoids cultured in the AEM hydrogels.

It was confirmed that, overall, all the markers were increased in expression level as the AEM concentration was increased. Based on the organoid formation efficiency and qPCR analysis result, the optimal AEM hydrogel concentration for small intestinal organoid culture was determined to be 4 mg/ml.

(B) Immunostaining of the small intestinal organoid cultured in the AEM hydrogel (4 mg/ml) was performed on day 6. As a result, it was confirmed that all of LGR5 related to stemness, ECAD related to tight junction, and MUC2 staining a goblet cell, one of the cells constituting the intestinal organoid, were well expressed at similar levels to those in the small intestinal organoid cultured in the Matrigel.

From the above results, it was confirmed that the formation and development of intestinal (small intestinal) organoids can be induced by three-dimensional culture using the decellularized AEM hydrogel.

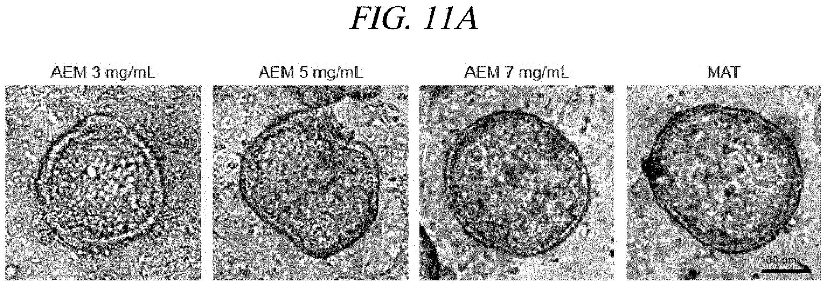

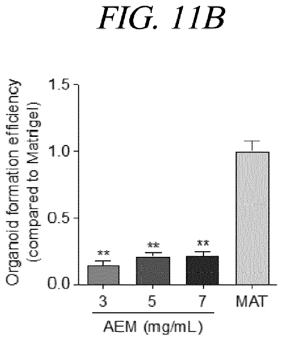

### Selection of optimal concentration of decellularized AEM hydrogel for lung organoid culture (FIG. 11A, FIG. 11B)

To select an optimal hydrogel concentration when applying the decellularized AEM hydrogel to lung organoid culture, a hydrogel was prepared at each AEM concentration, followed by lung organoid culture. The Matrigel (MAT) was used as a control group. Stem cells extracted from a lung tissue of a mouse were three-dimensionally cultured in the respective hydrogels to induce the formation of lung organoids. On day 7 of culture, the lung organoids formed at respective conditions were comparatively analyzed in terms of morphology and formation efficiency.
(A) It was confirmed that the lung organoids cultured in the AEM hydrogels at concentrations of 3 mg/ml, 5 mg/ml and 7 mg/ml, respectively, were formed with similar morphologies to that cultured in the Matrigel used as a control group.
(B) The lung organoids cultured in the AEM hydrogels at different concentrations (3mg/ml, 5 mg/ml and 7 mg/ml) and the Matrigel were compared in terms of formation efficiency. As a result, it was confirmed that the organoid formation efficiency was lower in the AEM hydrogels for all concentrations than in the Matrigel used as a control group.

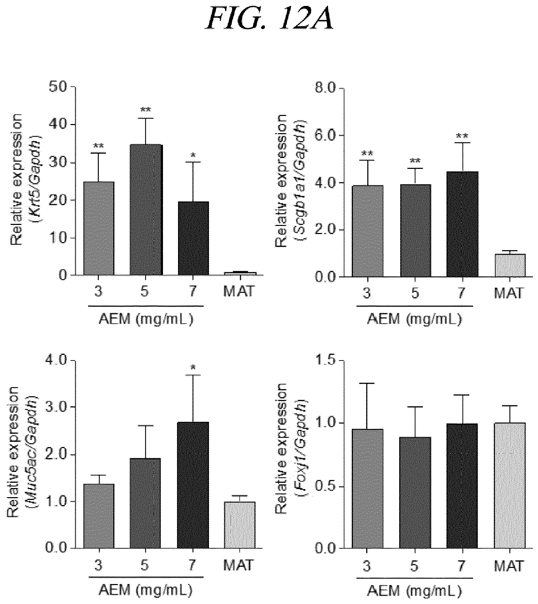

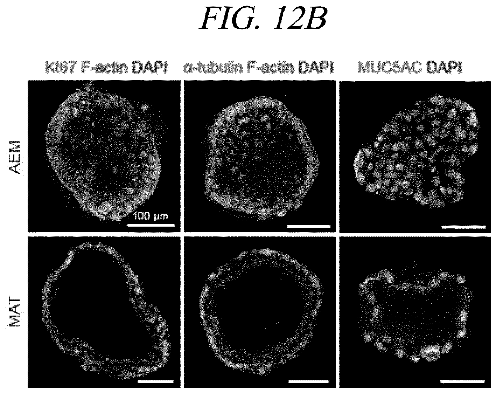

### Comparison of lung organoid culture patterns depending on concentration of decellularized AEM hydrogel (FIG. 12A, FIG. 12B)

The expression levels of four genes related to lung bronchioles were compared by quantitative PCR (qPCR) analysis. Lung organoids cultured for 7 days in the AEM hydrogels prepared at respective concentrations were compared with a lung organoid cultured in the Matrigel used as a control group. It was confirmed that Krt5, a basal cell-related gene, was expressed at least 19 times more in the AEM hydrogel groups for all concentrations than in the Matrigel group. Also, it was confirmed that Scgb1a1, a club cell-related gene, and Muc5ac, a goblet cell-related gene, were also expressed more in the AEM hydrogel groups for all concentrations. Foxj1, a ciliated cell-related gene, showed similar expression levels in the Matrigel group and the AEM hydrogel group. Based on the qPCR analysis result, 7 mg/ml was selected as the optimal AEM concentration and then applied to lung organoid culture.

In the organoids cultured in the AEM hydrogel (7 mg/ml) and the Matrigel, both goblet cell (MUC5AC) and ciliated cell (a-tubulin), cells present in a lung tissue, were observed. Also the expression of KI67, a protein related to cell proliferation, was observed, indicating proliferation of some cells in the lung organoids. Also, it was confirmed from cytoskeleton-related F-actin staining that cells in the organoid cultured in the AEM hydrogel formed an organically connected structure.

From the above results, it was confirmed that the use of the decellularized AEM hydrogel can promote the differentiation and development of lung organoids in spite of somewhat low lung organoid formation efficiency.

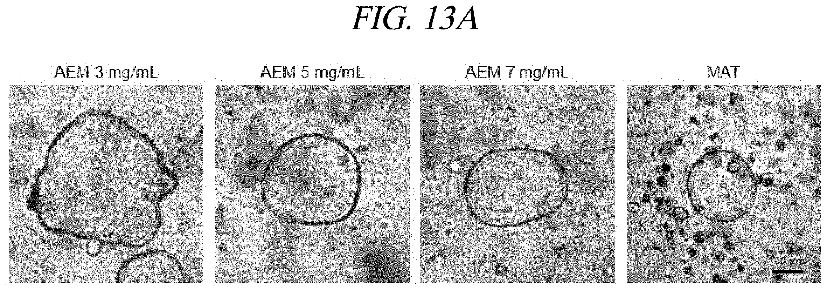

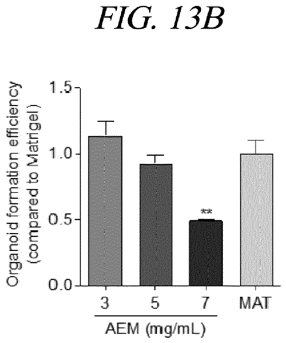

### Selection of optimal concentration of decellularized AEM hydrogel for pancreatic organoid culture (FIG. 13A, FIG. 13B)

To select an optimal scaffold concentration when applying the decellularized AEM hydrogel to pancreatic organoid culture, a hydrogel was prepared at each AEM concentration, followed by pancreatic organoid culture. Pancreatic duct cells extracted from a pancreas tissue were three-dimensionally cultured in the respective hydrogels to induce the formation of pancreatic organoids. On day 7 of culture, the pancreatic organoids formed at respective AEM concentrations were compared with the pancreatic organoid formed in the Matrigel (MAT) in terms of morphology.
(A) It was observed that pancreatic organoids were formed in the AEM hydrogels for all concentrations. As a result of analyzing the morphology and shape of the organoids, it was confirmed that the pancreatic organoids cultured in the AEM hydrogel scaffolds at concentrations of 5 mg/ml and 7 mg/ml grew with similar morphologies to that cultured in the Matrigel used as a control group.
(B) As a result of comparing the pancreatic organoids in terms of formation efficiency, there was no significant difference between culture using the AEM hydrogels (3 mg/ml and 5 mg/ml) and culture using the Matrigel. Thus, it was confirmed that human adipose tissue-derived AEM hydrogel can be applied as a substitute for Matrigel for pancreatic organoid culture.

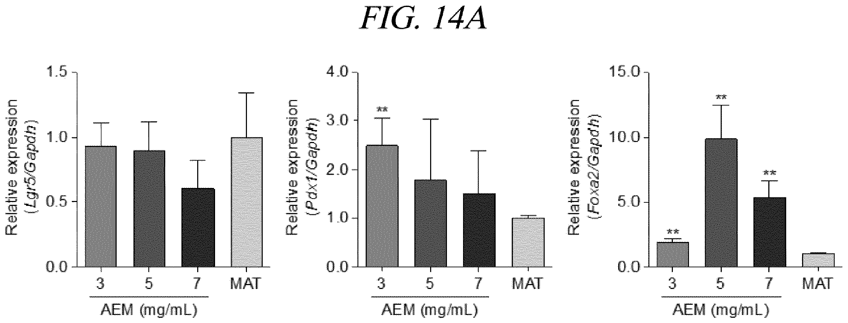

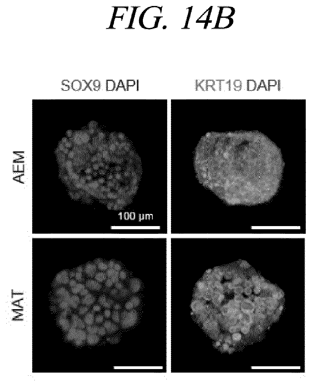

### Comparison of pancreatic organoid culture patterns depending on concentration of decellularized AEM hydrogel (FIG. 14A, FIG. 14B)

To select an optimal hydrogel concentration when applying the decellularized AEM hydrogel to pancreatic organoid culture, pancreatic organoids were cultured in AEM hydrogels prepared at three concentrations. The Matrigel (MAT) was used as a control group.
(A) As a result of comparing the gene expression levels of pancreatic organoids cultured for 7 days in the AEM hydrogels prepared at respective concentrations by qPCR analysis, it was confirmed that Lgr5, a stem ness-related gene, in the AEM hydrogel group showed a statistically similar expression level to that in the pancreatic organoid cultured in the Matrigel, and Pdx1 and Foxa2, pancreatic differentiation genes, significantly increased in the AEM hydrogel group. Based on the formation efficiency and qPCR analysis results, 5 mg/ml was selected as the optimal AEM hydrogel concentration for pancreatic organoid culture and then applied to pancreatic organoid culture.
(B) Immunostaining of the pancreatic organoids was performed on day 7 of culture to compare with the pancreatic organoid cultured in the Matrigel used as a control group in terms of pancreatic marker expression level. As a result, it was confirmed that SOX9 (pancreatic duct progenitor marker) and KRT19 (pancreatic duct marker), pancreatic tissue-specific markers, were well expressed in the pancreatic organoid cultured in the AEM hydrogel (5 mg/ml) at similar levels to those in the Matrigel group.

From the above results, it was verified that the formation and development of pancreatic organoids at a similar level to that of the Matrigel can be induced by three-dimensional culture using the decellularized AEM hydrogel.

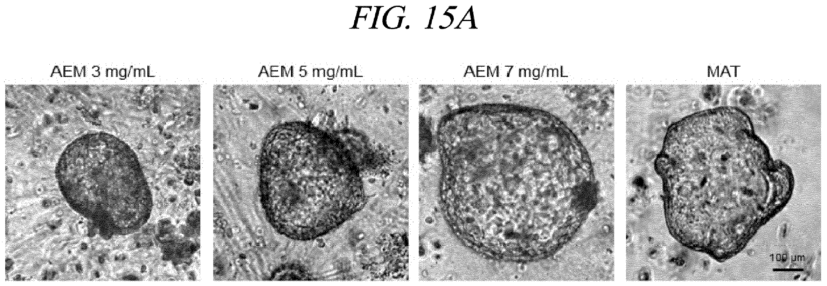

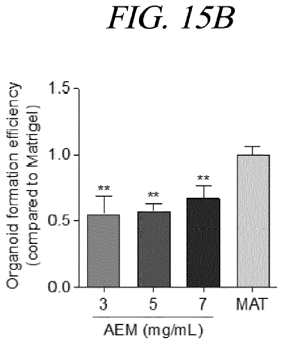

### Selection of optimal concentration of decellularized AEM hydrogel for gastric organoid culture (FIG. 15A, FIG. 15B)

To select an optimal hydrogel concentration when applying the decellularized AEM hydrogel to gastric organoid culture, a hydrogel was prepared at each AEM concentration, followed by gastric organoid culture. A stomach gland tissue, which is the most basic functional unit, was extracted from a stomach tissue of a mouse and then three-dimensionally cultured in the respective hydrogels to induce the formation of gastric organoids. On day 5 of culture, the gastric organoids formed at respective AEM concentrations were compared with the gastric organoid formed in the Matrigel (MAT) in terms of morphology and formation efficiency.
(A) It was confirmed that the gastric organoids cultured in the AEM hydrogels for all concentrations were formed with similar morphologies to that cultured in the Matrigel used as a control group.
(B) As a result of comparing the gastric organoids for respective AEM concentrations in terms of formation efficiency, the gastric organoid formation efficiency was lower in the AEM hydrogels for most of the concentrations than in the Matrigel, but highest in the AEM hydrogel with a concentration of 7 mg/ml among the AEM hydrogels.

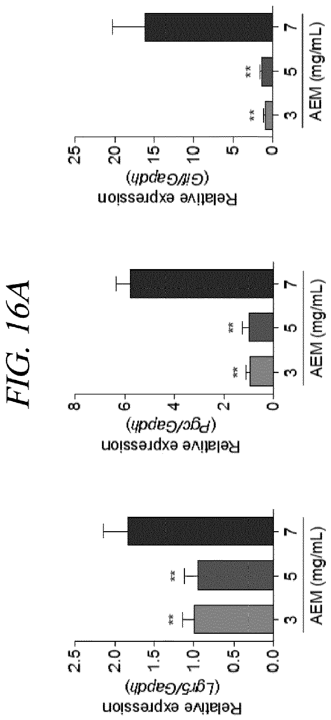

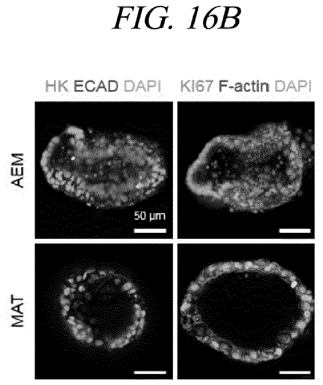

### Comparison of gastric organoid culture patterns depending on concentration of decellularized AEM hydrogel (FIG. 16A, FIG. 16B)

To select an optimal hydrogel concentration when applying the decellularized AEM hydrogel to gastric organoid culture, gastric organoids were cultured in hydrogels prepared at respective AEM concentrations.
(A) As a result of comparing the gene expression levels of gastric organoids cultured for 7 days in the AEM hydrogels prepared at respective concentrations by qPCR analysis, it was confirmed that Lgr5, a stem ness-related gene, and Pgc (chief cell) and Gif (parietal cell), genes expressed in specific cells of the stomach tissue, showed the highest expression levels in the AEM hydrogel group for a concentration of 7 mg/ml. Based on the organoid formation efficiency and qPCR results, the optimal AEM hydrogel concentration for gastric organoid culture was determined to be 7 mg/ml.
(B) Likewise, immunostaining of the gastric organoid cultured in the AEM hydrogel (7 mg/ml) was performed on day 5. As a result, it was confirmed that various markers, such as HK (parietal cell), a stomach tissue cell marker, KI67 related to stemness, ECAD related to tight junction, and F-actin forming cytoskeleton, were well expressed in the AEM hydrogel at similar levels to those in the Matrigel group.

From the above results, it was confirmed that the formation and development of gastric organoids can be induced by three-dimensional culture using the decellularized AEM hydrogel.

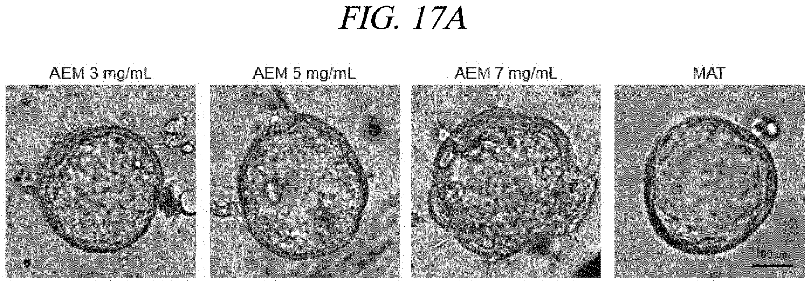

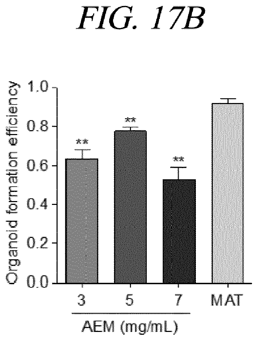

### Selection of optimal concentration of decellularized AEM hydrogel for kidney organoid culture (FIG. 17A, FIG. 17B)

To select an optimal hydrogel concentration when applying the decellularized AEM hydrogel to kidney organoid culture, a hydrogel was prepared at each AEM concentration, followed by kidney organoid culture. The renal tubular fragment of a mouse was extracted and then three-dimensionally cultured in the AEM hydrogels for respective concentrations to induce the formation of kidney organoids. Subculture was carried out on day 7 of culture and further cultured for 5 days to check the morphology and formation efficiency of the kidney organoids formed at respective AEM concentrations on day 12 of culture. The Matrigel (MAT) was used as a control group.
(A) It was confirmed that the kidney organoids were formed in the AEM hydrogels for all concentrations, and particularly, the kidney organoid cultured at a concentration of 5 mg/ml was formed with the most similar morphology to that cultured in the Matrigel used as a control group.
(B) The number of organoids was measured immediately after subculture (day 0) and on day 5 based on the time of subculture and expressed as a ratio to measure the formation efficiency. It was confirmed that, overall, the AEM hydrogels showed lower formation efficiency than the Matrigel, but the AEM hydrogel with a concentration of 5 mg/ml showed the highest formation efficiency.

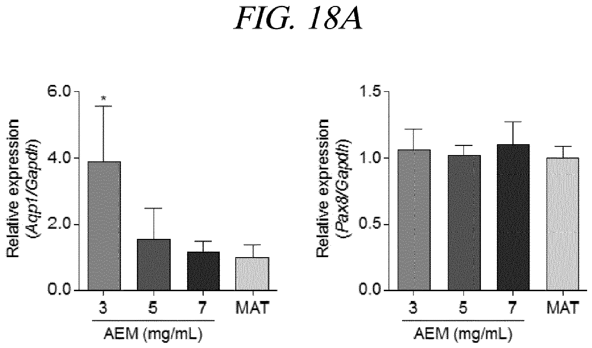

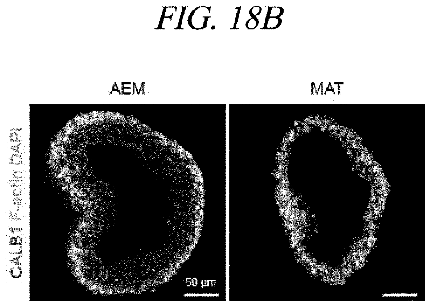

### Comparison of kidney organoid culture patterns depending on concentration of decellularized AEM hydrogel (FIG. 18A, FIG. 18B)

To select an optimal hydrogel concentration when applying the decellularized AEM hydrogel to kidney organoid culture, kidney organoids were cultured in hydrogels prepared at respective AEM concentrations. The Matrigel (MAT) was used as a control group.
(A) As a result of comparing the gene expression levels of kidney organoids cultured for 12 days in the AEM hydrogels prepared at respective concentrations by qPCR analysis, Aqp1 (proximal tubule cell), a gene expressed in specific cells of the kidney, showed a decrease in expression level as the AEM concentration increased, but it was confirmed that the expression level of Aqp1 was higher in the AEM hydrogels for all concentrations than in the Matrigel group. Also, it was confirmed that, overall, Pax8 (renal progenitor cell) related to stemness showed a similar expression level to that of the Matrigel group. Based on the organoid formation efficiency, organoid morphology and qPCR results, the optimal AEM hydrogel concentration for kidney organoid culture was determined to be 5 mg/ml.
(B) Immunostaining was performed on day 12 of culture to analyze expression of markers. As a result, it was confirmed that CALB1 (distal tubule cell), a kidney-specific differentiation marker, was well expressed in the kidney organoids cultured in the AEM hydrogel (5 mg/ml) at a similar level to that in the organoid cultured in the Matrigel used as a control group. Also, it was confirmed from cytoskeleton-related F-actin staining that cells in the kidney organoid cultured in the AEM hydrogel formed an organically connected structure.

From the above results, it was confirmed that the formation and development of kidney organoids can be induced by three-dimensional culture using the decellularized AEM hydrogel.

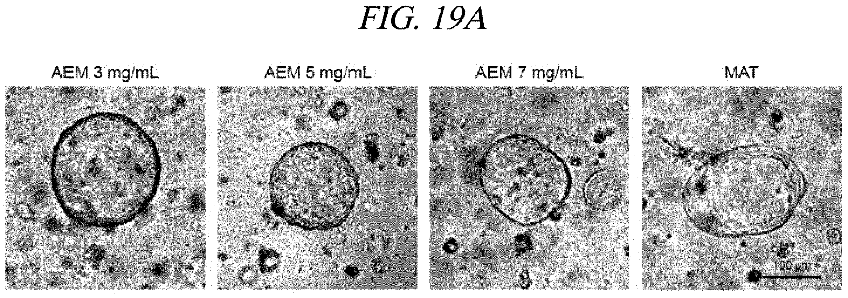

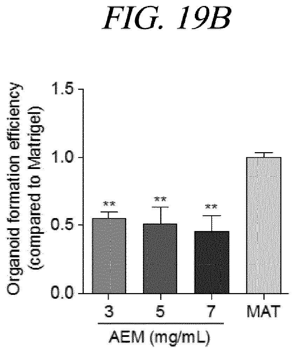

### Selection of optimal concentration of decellularized AEM hydrogel for hepatic (biliary) organoid culture (FIG. 19A, FIG. 19B)

To select an optimal hydrogel concentration when applying the decellularized AEM hydrogel to hepatic (biliary) organoid culture, a hydrogel was prepared at each AEM concentration, followed by hepatic (biliary) organoid culture. Biliary cells extracted from a hepatic tissue of a mouse were three-dimensionally cultured in the AEM hydrogels for respective concentrations to induce the formation of hepatic organoids. On day 7 of culture, the hepatic organoids formed at respective AEM conditions were compared with the hepatic organoid formed in the Matrigel (MAT) in terms of morphology and formation efficiency.
(A) It was confirmed that the hepatic organoids cultured in the AEM hydrogels for all concentrations were formed with similar morphologies to that cultured in the Matrigel used as a control group.
(B) As a result of comparing the hepatic organoids for respective AEM concentrations in terms of formation efficiency, the hepatic organoid formation efficiency was lower in the AEM hydrogels for most of the concentrations than in the Matrigel, but highest in the AEM hydrogel with a concentration of 3 mg/ml among the AEM hydrogels.

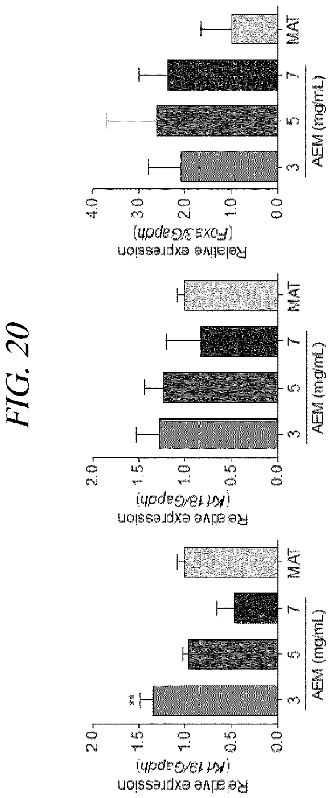

### Comparison of hepatic (biliary) organoid culture patterns depending on concentration of decellularized AEM hydrogel (FIG. 20)

To select an optimal hydrogel concentration when applying the decellularized AEM hydrogel to hepatic (biliary) organoid culture, hepatic organoids were cultured in hydrogels prepared at respective AEM concentrations. The Matrigel (MAT) was used as a control group.

As a result of comparing the gene expression levels of hepatic organoids cultured for 7 days in the AEM hydrogels prepared at respective concentrations by qPCR analysis, it was confirmed that Krt19, a biliary marker, and Krt18, a liver differentiation marker, among differentiation-related markers were expressed in the AEM hydrogels for most of the concentrations except 7 mg/ml at similar or higher expression levels than those in the Matrigel group, and Foxa3, a liver differentiation marker, showed an increase in expression level in all of the AEM hydrogel group compared with the Matrigel group. Based on the above results, the optimal AEM hydrogel concentration for hepatic organoid culture was determined to be 3 mg/ml.

From the above results, it was confirmed that the formation and development of hepatic (biliary) organoids can be induced by three-dimensional culture using the decellularized AEM hydrogel.

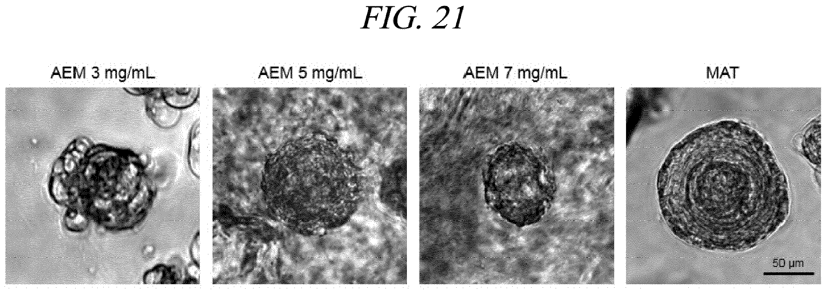

### Selection of optimal concentration of decellularized AEM hydrogel for esophageal organoid culture (FIG. 21)

To select an optimal scaffold concentration when applying the decellularized AEM hydrogel to esophageal organoid culture, a hydrogel was prepared at each AEM concentration, followed by esophageal organoid culture.

The esophageal muscle layer of a mouse was removed, followed by enzyme treatment to extract stem cells. Then, the stem cells were cultured in the AEM hydrogels. As a result of comparing the esophageal organoids formed at respective AEM concentrations with the esophageal organoid formed in the Matrigel (MAT) in terms of morphology on day 9 of culture, it was confirmed that the esophageal organoid was formed in the AEM hydrogel with a concentration of 5 mg/ml with the most similar morphology to that in the Matrigel.

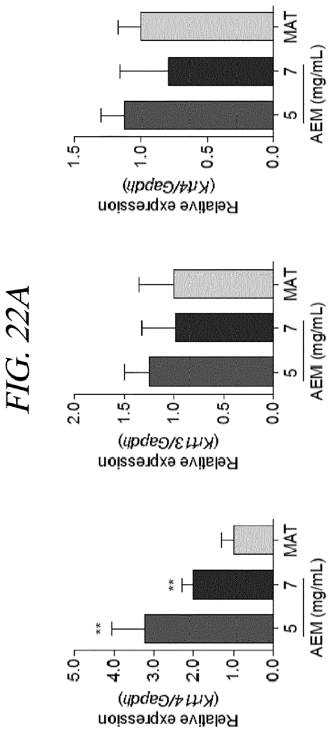

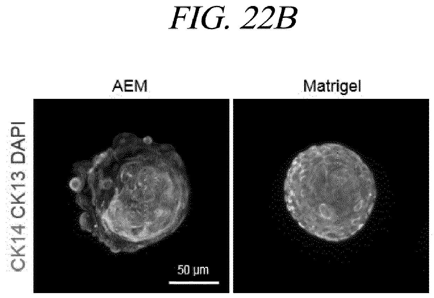

### Comparison of esophageal organoid culture patterns depending on concentration of decellularized AEM hydrogel (FIG. 22A, FIG. 22B)

To select an optimal hydrogel concentration when applying the decellularized AEM hydrogel to esophageal organoid culture, esophageal organoids were cultured in hydrogels prepared at respective AEM concentrations. The Matrigel (MAT) was used as a control group.
(A) As a result of comparing the gene expression levels of esophageal organoids cultured for 9 days in the AEM hydrogels prepared at two concentrations by qPCR analysis, it was confirmed that the expression level of Krt14, a gene expressed in the esophageal basal layer, significantly increased in the AEM hydrogel groups for all concentrations compared with the Matrigel group, and the expression levels of Krt13 and Krt4 expressed in the suprabasal layer was higher in the AEM hydrogel group for a concentration of 5 mg/ml than in the Matrigel group. Based on the above results, the optimal AEM hydrogel concentration for esophageal organoid culture was determined to be 5 mg/ml.
(B) Immunostaining was performed on day 9 of culture to analyze expression of markers. As a result, it was confirmed that cytokeratin 14 (CK14), a protein expressed in the basal layer, was well expressed in the esophageal organoid cultured in the AEM hydrogel (5 mg/ml) at a similar level to that in the organoid cultured in the Matrigel used as a control group. Also, it was confirmed that cytokeratin 13 (CK13), a protein expressed in the suprabasal layer, was expressed at a similar level in both groups.

From the above results, it was confirmed that the formation and development of esophageal organoids can be induced by three-dimensional culture using the decellularized AEM hydrogel.

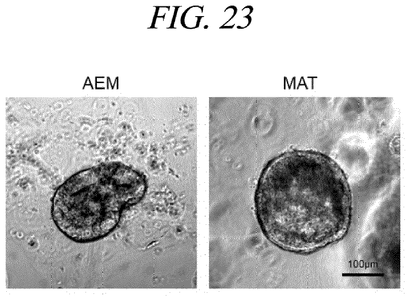

### Intestinal (colonic) organoid culture using decellularized AEM hydrogel (FIG. 23)

A colonic organoid was cultured in the decellularized AEM hydrogel. The colon of a mouse was obtained, and the colonic crypt was isolated to obtain colonic stem cells. Then, the colonic stem cells were three-dimensionally cultured in the Matrigel and the AEM hydrogel with a concentration of 5 mg/ml to induce the formation of colonic organoids. On day 7 of culture, the colonic organoids formed in the Matrigel and the AEM hydrogel were compared in terms of organoid morphology.

It was confirmed that the colonic organoid cultured in the AEM hydrogel was formed with a similar morphology to that cultured in the Matrigel used as a control group.

From the above results, it was confirmed that the formation and development of intestinal (colonic) organoids can be induced by three-dimensional culture using the decellularized AEM hydrogel.

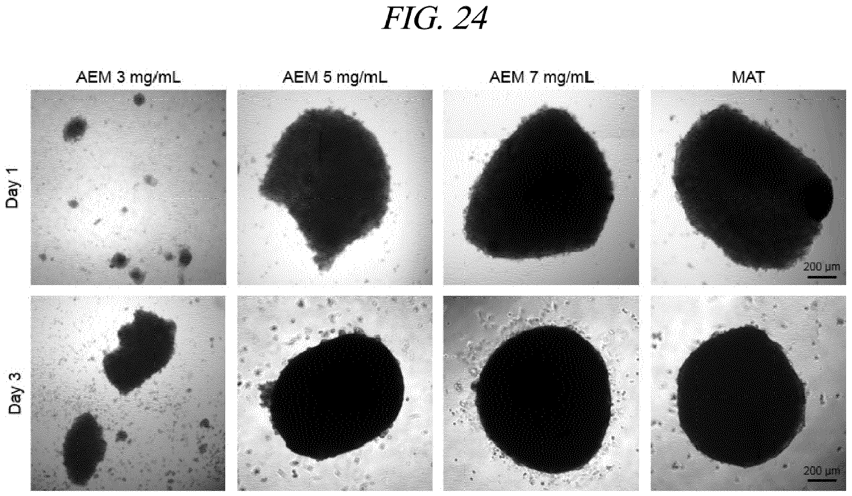

### Selection of optimal concentration of decellularized AEM hydrogel for human-induced pluripotent stem cell (hiPSC)-derived hepatic organoid culture (FIG. 24)

To select an optimal scaffold concentration when applying the decellularized AEM hydrogel to human-induced pluripotent stem cell (hiPSC)-derived hepatic organoid culture, a hydrogel was prepared at each AEM concentration, followed by hiPSC-derived hepatic organoid culture.

Cells required for hepatic organoid formation from hiPSCs (hiPSC-derived hepatic endodermal cells, vascular endothelial cells and mesenchymal stem cells) were cultured in the AEM hydrogels at a ratio of 10:7:2. The cells were aggregated in 24 hours, forming hepatic organoids. After 3 days, the organoids were condensed more densely and grew agglomerated.

As a result of analyzing the morphology and shape of the organoids, it was confirmed that the hiPSC-derived hepatic organoids cultured in the AEM hydrogel scaffolds at concentrations of 5 mg/ml and 7 mg/ml grew with the most similar morphologies to that cultured in the Matrigel used as a control group.

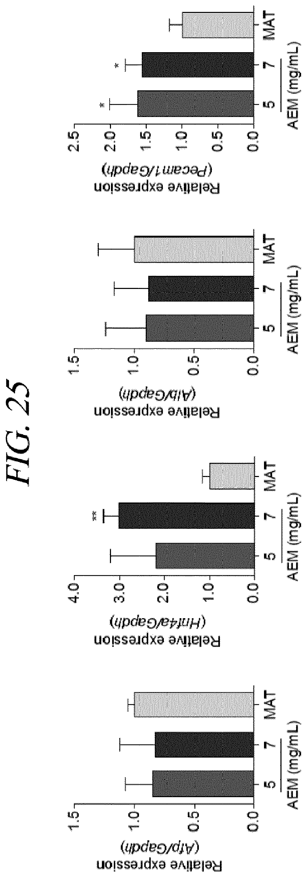

### Comparison of hiPSC-derived hepatic organoid culture patterns depending on concentration of decellularized AEM hydrogel (FIG. 25)

As a result of comparing the gene expression levels of hiPSC-derived hepatic organoids cultured for 5 days in the AEM hydrogels prepared at two concentrations by qPCR analysis, it was confirmed that Afp, an early hepatocyte marker, in the AEM hydrogel group showed a statistically similar expression level to that in the hepatic organoid cultured in the Matrigel, whereas Hnf4a showed a higher expression level in the AEM hydrogel group. Also, it was confirmed that Alb, mature hepatocyte marker, in the AEM hydrogel group showed a similar expression level to that in the Matrigel group, and Pecam1, a vascular-related marker, showed a high expression level in the organoids cultured in the AEM hydrogels.

Therefore, based on the organoid morphology and qPCR results, the optimal AEM hydrogel concentration for hiPSC-derived hepatic organoid culture was determined to be 7 mg/ml.

From the above results, it was verified that the formation and development of hiPSC-derived hepatic organoids at a similar level to that of the Matrigel can be induced by three-dimensional culture using the decellularized AEM hydrogel.

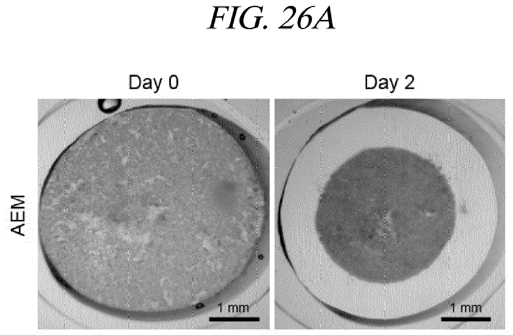

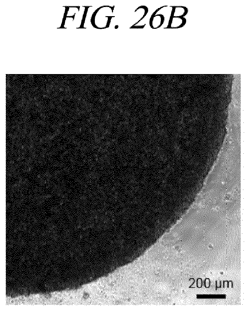

### Cardiac organoid culture using decellularized AEM hydrogel (FIG. 26A, FIG. 26B)

Cardiomyocytes derived from hiPSCs were three-dimensionally cultured in the decellularized AEM hydrogel to prepare a cardiac organoid. To this end, 3×10⁵ cardiomyocytes were encapsulated in 15 µL of the AEM hydrogel with a concentration of 7 mg/ml and cultured for 2 days.
(A) As a result, it was confirmed that the size of the AEM hydrogel decreased with contraction of the AEM hydrogel on day 2 of culture, and the cardiomyocytes formed a structured cardiac organoid.
(B) It was confirmed that the cardiac organoid has a tissue-like structure strongly bonded to each other due to interactions among the cardiomyocytes.

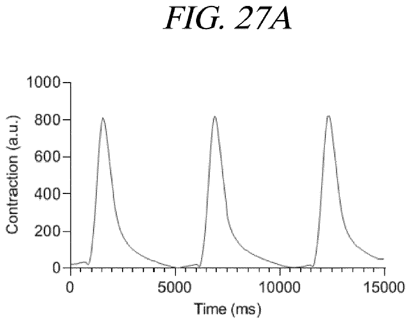

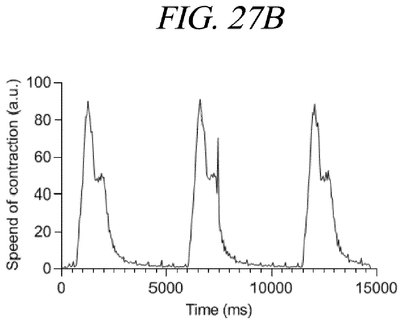

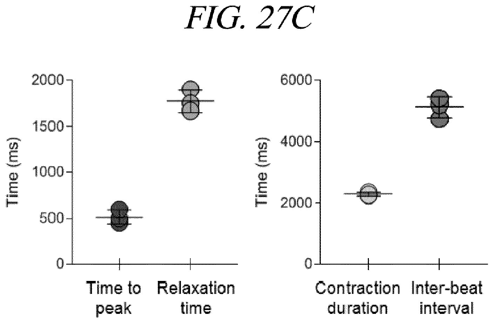

### Analysis of functionality of cardiac organoid prepared in decellularized AEM hydrogel (FIG. 27A, FIG. 27B, FIG. 27C)

Spontaneous contraction of a hiPSC-derived cardiac organoid prepared in the decellularized AEM hydrogel was analyzed.

A quantitative analysis of the cardiac organoid in terms of spontaneous contraction intensity (A) and speed of contraction (B) for 15 seconds confirmed regular contractions.

(C) As a result of quantitative analysis of time to peak, relaxation time, contraction duration and inter-beat interval for analysis related to contraction, which is the main function of a myocardial tissue, it was confirmed that the cardiac organoid prepared in the AEM hydrogel can implement similar functions to a cardiac tissue.

From the above results, it was verified that the formation and functional development of hiPSC-derived cardiac organoids can be induced by three-dimensional culture using the decellularized AEM hydrogel.

The present disclosure has been described with reference to the preferred exemplary embodiments thereof. It can be understood by a person with ordinary skill in the art that the present disclosure can be implemented as being modified and changed within the scope departing from the spirit and the scope of the present disclosure. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. A scaffold for culture and transplantation of an organoid using an adipose extracellular matrix (AEM).

2. The scaffold of Claim 1,
wherein the AEM is prepared by using a mixed solution of Triton X-100 and ammonium hydroxide.

3. The scaffold of Claim 1,
wherein a concentration of the AEM in the scaffold is from 1 mg/ml to 10 mg/ml.

4. A method of preparing a scaffold for culture and transplantation of an organoid, comprising:
a process (1) of crushing an isolated adipose tissue; and
a process (2) of treating the crushed adipose tissue with Triton X-100 and ammonium hydroxide for decellularization to prepare a decellularized AEM.

5. The method of preparing a scaffold for culture and transplantation of an organoid of Claim 4, further comprising:
after the process (2), a process (3) of lyophilizing the decellularized AEM to prepare a lyophilized AEM.

6. The method of preparing a scaffold for culture and transplantation of an organoid of Claim 5, further comprising:
after the process (3), a process (4) of forming a scaffold for culture and transplantation of an organoid in the form of a hydrogel with the lyophilized AEM.

7. The method of preparing a scaffold for culture and transplantation of an organoid of Claim 6,
wherein in the process (4), the lyophilized AEM is dissolved in a pepsin solution and the pH of the solution is adjusted to form the hydrogel.

8. A method of culturing an organoid in the scaffold of Claim 1 or a scaffold prepared by the preparation method of Claim 4.
